# EUROPEAN PATENT SPECIFICATION

(11) **EP 3 809 947 B1**
(45) Date of publication and mention of the grant of the patent: **04.11.2026**
(21) Application number: 20770049.3
(22) Date of filing: 13.03.2020
(51) Int. Cl.: A61B 1/00, A61B 1/267, A61B 1/05, A61B 1/06, A61B 1/07

(54) **UNIVERSAL LARYNGOSCOPE BLADE**
UNIVERSELLE LARYNGOSKOPKLINGE
LAME DE LARYNGOSCOPE UNIVERSELLE

(30) Priority: 14.03.2019 US 201962818484 P
(43) Date of publication of application: 28.04.2021
(73) Proprietor: Teleflex Medical LLC, Morrisville, North Carolina 27560 (US)
(72) Inventor: ELBAZ, Aviram, Apex, North Carolina 27502 (US); DUBOIS, Nate, Durham, North Carolina 27703 (US)
(74) Representative: Carpmaels & Ransford LLP
(86) International application number: PCT/US2020/022564
(87) International publication number: WO 2020/186141

(56) References cited:
- GB-A- 2 491 189
- GB-A- 2 491 189
- US-A1- 2011 245 609
- US-A1- 2013 018 227
- US-A1- 2017 202 447
- US-A1- 2018 008 137
- US-B1- 7 824 331
- US-B1- 7 824 331

## Description

### Cross-Reference to Related Applications

This application claims priority to U.S. Provisional Patent Application No. 62/818,484, filed March 14, 2019.

### Technical Field

The invention relates to the field of laryngoscopes in general, and particularly for universal laryngoscope blades.

### Background

Laryngoscopes typically include a lamp that emits light to illuminate a field of view during intubation. The lamp for illuminating the larynx during use is attached either to the laryngoscope blade, or is provided in the handle and the blade includes a light guide that guides the light produced in the handle. Known laryngoscope blades that include the lamp are referred to herein as conventional blades. Known laryngoscope blades that include a light guide for guiding light produced from a lamp provided in the handle are referred to herein as fiber-illuminated blades.

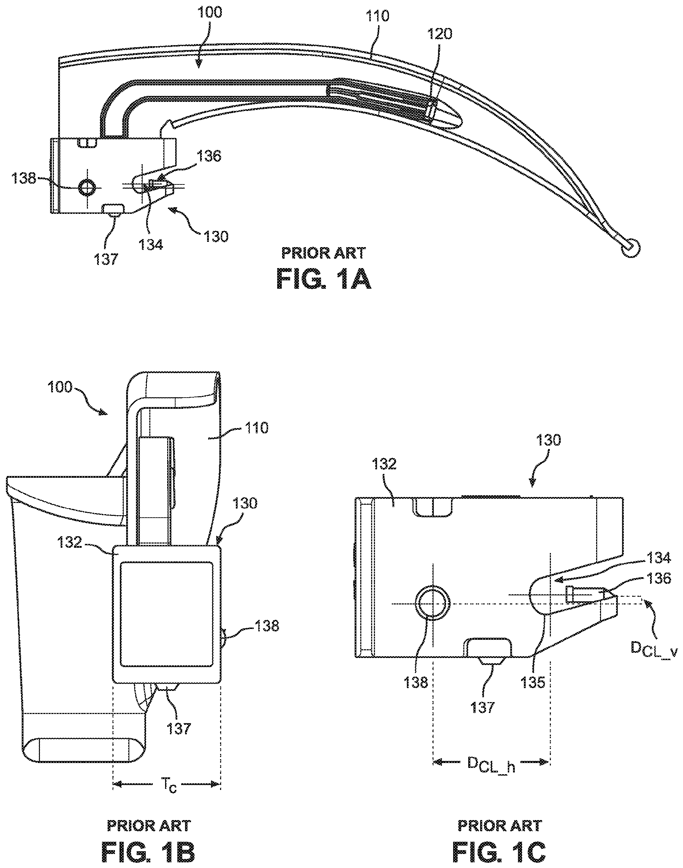

FIGS. 1A-C show views of an exemplary known conventional blade 100. FIG. 1A shows a side view of the conventional blade 100. FIG. 1B shows a rear view of the convention blade. FIG. 1C shows a magnified side view of the blade fitting 130 of the conventional blade 100. Unless otherwise indicated, direction terms such as "top," "bottom," "front," "rear," "above," "below," "vertical," "horizontal," etc., when used to describe the position of a structure shown in the figures, are directed to the respective direction of the structure as oriented in the figures. For example, the blade fitting 130 of the conventional blade 100 (described in further detail below) is provided on the bottom of the conventional blade 100, as oriented in FIGS. 1A-C. Nevertheless, the direction terms used herein do not necessarily correspond to the orientation of the structure with respect to a user or a patient during intended use.

The conventional blade 100 includes a blade body 110, a lamp 120, and a blade fitting 130. The blade fitting 130 includes a body 132, a lock, an electrical contact 137, and a hinge slot 134. As shown in FIG. 1B, the body 132 has a thickness Tc. The lock includes a hinge lock 136 disposed within a bottom surface of the hinge slot 134. The lock also includes a side lock 138 provided on a side of the body 132. A center point of the side lock 138 is indicted at the intersection of side lock crosshairs, as shown in FIG. 1C. The hinge lock 136 and the side lock 138 are detent mechanisms. The electrical contact 137 is provided on a bottom of the body 132. The electrical contact 137 is in electrical communication with the lamp 120 via one or more wires that supply power to the lamp 120. The hinge slot 134 extends into the body 132 at an angle from a front the body 132 towards a central region of the body 132 and terminates at a bearing surface 135. The bearing surface 135 has a semicircular shape having a diameter and a virtual center point, indicated at the intersection of hinge slot crosshairs shown in FIG. 1C, about which the bearing surface 135 forms the semicircular shape.

With reference to the magnified side view of the blade fitting 130 of the conventional blade 100 shown in FIG. 1C, the center point of the side lock 138 is spaced from the virtual center point of the semicircular bearing surface 135 by a distance having a horizontal distance component D_{CL_h}, and a vertical distance component D_{CL_v}. The center point of the side lock 138 is spaced rearwardly from the virtual center point of the semicircular bearing surface 135 by the horizontal distance component D_{CL_h}, and is spaced below the virtual center point of the bearing surface 135 by the vertical distance component D_{CL_v}.

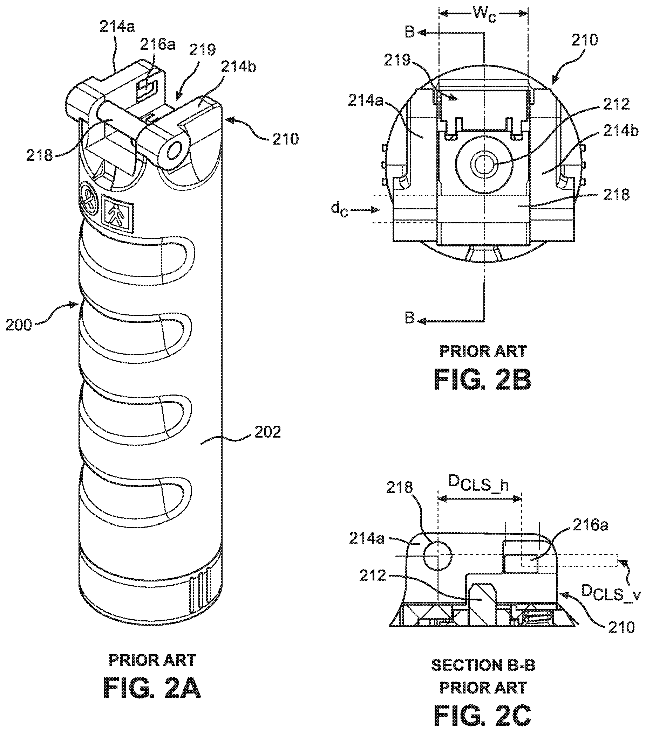

FIGS. 2A-C show views of an exemplary known conventional handle 200. FIG. 2A shows a perspective view of the conventional handle 200. FIG. 2B shows a top view of a hook-on fitting 210 of the conventional handle 200. FIG. 2C shows a cut-away side view of the hook-on fitting 210 of the conventional handle 200 taken along section B-B of FIG. 2B.

The conventional handle 200 includes a grip 202 and a hook-on fitting 210 disposed at a top of the grip 202. The grip 202 is configured to be grasped by the hand of a user of the laryngoscope. The conventional handle 200 also includes one or more batteries disposed within an interior of the grip 202. The one or more batteries serve as a power source for the lamp 120 of the conventional blade 100.

The hook-on fitting 210 of the conventional handle 200 is specifically designed for attachment to the blade fitting 130 of the conventional blade 100, and *vice versa.* The hook-on fitting 210 includes an electrical contact 212, first and second side supports 214a, 214b, and a hinge pin 218. The electrical contact 212 is in electrical communication with the batteries via one or more wires that supply power to the electrical contact 212. The electrical contact 212 is configured to supply power to the electrical contact 137 of the blade fitting 130 of the conventional blade 100 when the conventional blade 100 is engaged with the conventional handle 200 in an operating position.

The first and second side supports 214a, 214b extend vertically from the top of the grip 202. The first and second side supports 214a, 214b extend parallel to each other and are spaced apart from each other. The space bounded within the top of the grip 202 and the first and second side supports 214a, 214b forms a seat 219. The seat 219 has a width W_{C} between the first and second side supports 214a, 214b. The width W_{C} is greater than the thickness Tc of the body 132 of the blade fitting 130 such that the seat 219 is configured to receive the body 132 of the blade fitting 130 therein.

The hinge pin 218 is attached to (i.e., extends through) the front of each of the first and second side supports 214a, 214b and extends across the seat 219. The hinge pin 218 is a cylinder having a diameter dc. The diameter d_{C} is less than the diameter of the semicircular bearing surface 135 of the blade fitting 130 such that the hinge pin 218 is configured to be received within, and be rotatably supported by, the hinge slot 134 of the conventional blade 100. The hinge lock 136 is configured to lock the hinge pin 218 within the hinge slot 134.

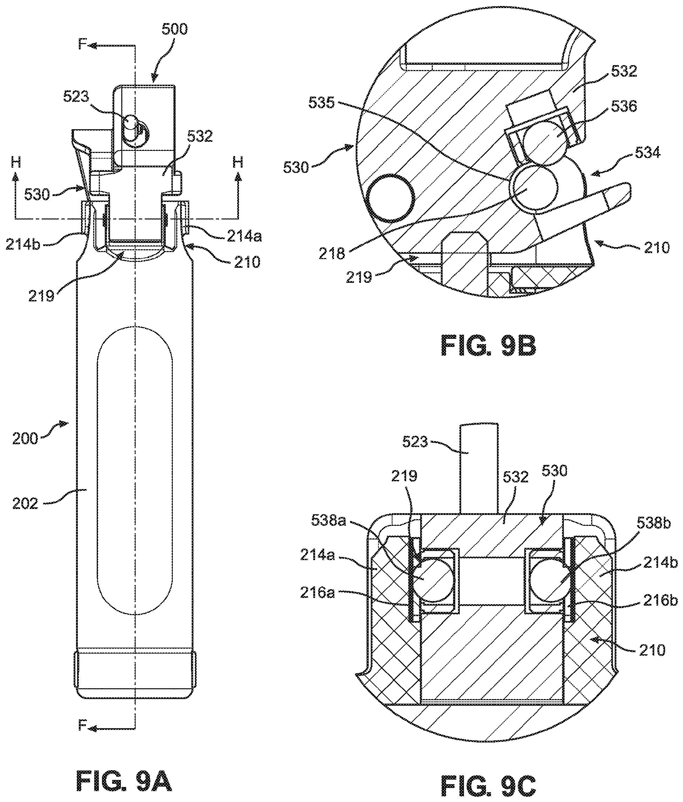

At least one of the side supports includes a lock slot protruding into a seat-facing side of the respective side support. For example, the first side support 214a includes a first lock slot 216a and the second side support 214b includes a second lock slot 216b (shown in FIG. 9C). The lock slot is configured to receive the side lock 138 of the blade fitting 130 to hold the body 132 within the seat 219. To ensure that the side lock 138 fits within the lock slot when the conventional blade 100 is engaged with the conventional handle 200, a center of each respective lock slot is spaced from a center point of the hinge pin 218, designated by the intersection of the hinge pin crosshairs shown in FIG. 2C, by a distance having a horizontal distance component D_{CLS_h}, and a vertical distance component D_{CLS_v}. The center of each respective lock slot is spaced rearwardly from the center point of the hinge pin 218 by the horizontal distance component D_{CLS_h}, and is spaced below the center point of the hinge pin 218 by the vertical distance component D_{CLS_v}.

The blade fitting 130 of the conventional blade 100 is configured to engage with hook-on fitting 210 of the conventional handle 200 to lock the conventional blade 100 within the conventional handle 200 in an operating position, in which the laryngoscope is ready-for-use. To place the conventional blade 100 in the operating position, the blade fitting 130 is first attached to the hook-on fitting 210. The blade fitting 130 is attached to the hook-on fitting 210 by placing the hinge pin 218 of the hook-on fitting 210 within the hinge slot 134 of the blade fitting 130 and providing relative movement between the hinge pin 218 and the hinge slot 134 until the hinge pin 218 abuts against the semicircular bearing surface 135 of the hinge slot 134 and is locked therein by the hinge lock 136. Relative rotational movement is provided between the conventional blade 100 and the conventional handle 200 such that the hinge slot 134 and the hinge pin 218 are rotated relative to one and other until the body 132 of the blade fitting 130 slides within the seat 219 of the hook-on fitting 210. After the body 132 of the blade fitting 130 is fully received within the seat 219 of the hook-on fitting 210, the side lock 138 of the blade fitting 130 engages with the lock slot of the hook-on fitting 210. The conventional blade 100 is thus locked within the conventional handle 200 in the operating position. In the operating position, the electrical contact 137 of the blade fitting 130 is engaged with the electrical contact 212 of the hook-on fitting 210, and the laryngoscope is ready-for-use.

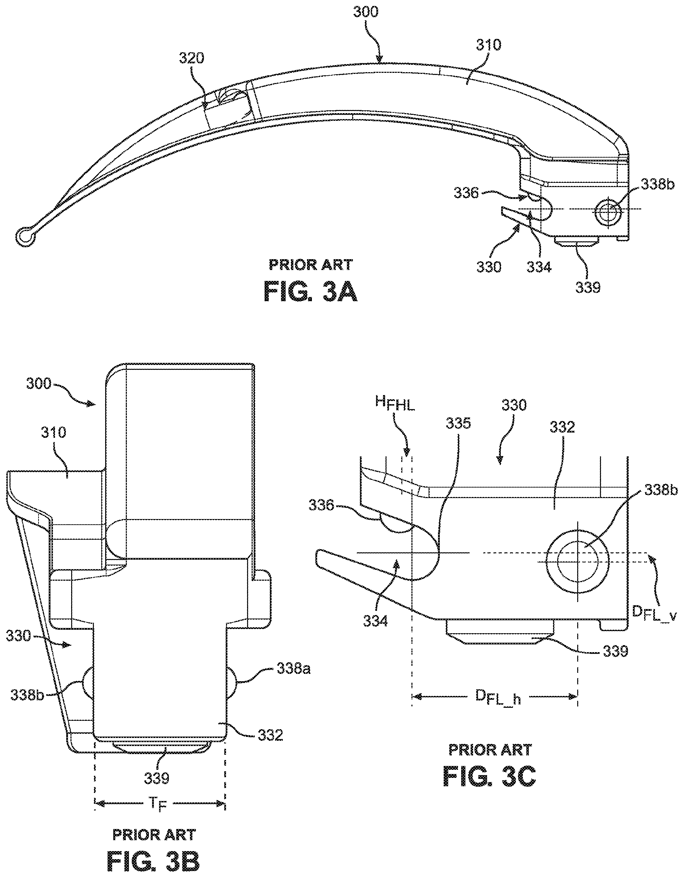

FIGS. 3A-C show views of an exemplary known fiber-illuminated blade 300. Fig. 3A shows a side view of the fiber-illuminated blade 300. FIG. 3B shows a rear view of the fiber-illuminated blade 300. FIG. 3C shows a magnified side view of the blade fitting 330 of the fiber-illuminated blade 300.

The fiber-illuminated blade 300 includes a blade body 310, a light guide 320, and a blade fitting 330. The blade fitting 330 includes a body 332, a lock, a light guide interface 339, and a hinge slot 334. As shown in FIG. 3B, the body 332 has a thickness T_{F}. The lock includes a hinge lock 336 disposed within a top surface of the hinge slot 334. The lock also includes first and second side locks 338a, 338b respectively provided on opposite sides of the body 332. A center point of the second side lock 338b is indicted at the intersection of side lock crosshairs, as shown in FIG. 3C. The hinge lock 336 and the first and second side locks 338a, 338b are detent mechanisms. The light guide interface 339 is an end of the light guide 320 that is provided on a bottom of the body 332. The light guide interface 339 is configured to interface with an optical pathway 412 of a fiber-illuminated handle 400 (described below) to receive light transmitted from a lamp disposed within the fiber-illuminated handle 400. The hinge slot 334 extends into the body 332 at an angle from a front the body 332 towards a central region of the body 332 and terminates at a bearing surface 335. The bearing surface 335 has semicircular shape having a diameter and a virtual center point, indicated by the intersection of hinge slot crosshairs shown in FIG. 3C, about which the bearing surface 335 forms the semicircular shape.

With reference to the magnified side view of the blade fitting 330 of the fiber-illuminated blade 300 shown in FIG. 3C, a center of the hinge lock 336 is spaced forwardly from the virtual center of the semicircular bearing surface 335 by a horizontal distance H_{FHL}. The center point of the second side lock 338b is spaced from the virtual center point of the semicircular bearing surface 335 by a distance having a horizontal distance component D_{FL_h}, and a vertical distance component D_{FL_v}. The center point of the second side lock 338b is spaced rearwardly from the virtual center point of the semicircular bearing surface 335 by the horizontal distance component D_{FL_h}, and is spaced below the virtual center point of the bearing surface 335 by the vertical distance component D_{FL_v}. A center point of the first side lock 338b is provided at a corresponding position at the other side surface of the body 332 with respect to the virtual center point of the semicircular bearing surface 335 such that the first and second side locks 338a, 338b are spaced the same distance from the virtual center point of the semicircular bearing surface 335.

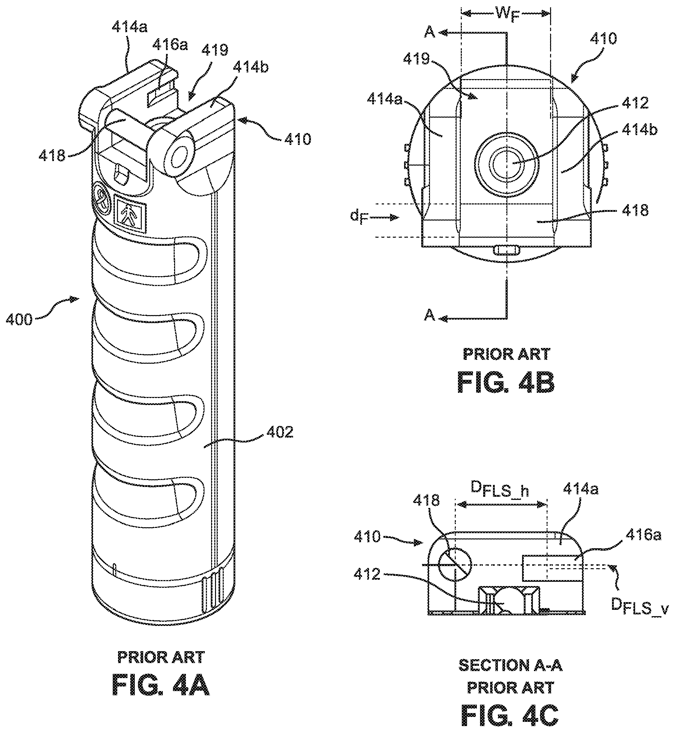

FIGS. 4A-C show views of an exemplary known fiber-illuminated handle 400. FIG. 4A shows a perspective view of the fiber-illuminated handle 400. FIG. 4B shows a top view of the hook-on fitting 410 of the fiber-illuminated handle 400. FIG. 4C shows a cut-away side view of the hook-on fitting 410 of the fiber-illuminated handle 400 taken along section A-A of FIG. 4B.

The fiber-illuminated handle 400 includes a grip 402 and a hook-on fitting 410 disposed at a top of the grip 402. The grip 402 is configured to be grasped by the hand of a user of the laryngoscope. The fiber-illuminated handle 400 also includes one or more batteries, the lamp, and an optical pathway 412. The one or more batteries serve as a power source for the lamp provided within the fiber-illuminated handle 400. The optical pathway 412 supplies light, emitted from the lamp, to the light guide interface 339 of the fiber-illuminated blade 300 when the fiber-illuminated blade 300 is connected to the fiber-illuminated handle 400 in an operating position, described below.

The hook-on fitting 410 of the fiber-illuminated handle 400 is specifically designed for attachment to the blade fitting 330 of the fiber-illuminated blade 300, and *vice versa.* The hook-on fitting 410 includes an end of the optical pathway 412, first and second side supports 414a, 414b, and a hinge pin 418. The first and second side supports 414a, 414b extend vertically from the top of the grip 402. The first and second side supports 414a, 414b extend parallel to each other and are spaced apart from each other. The space bounded within the top of the grip 402 and the first and second side supports 414a, 414b forms a seat 419. The seat 419 has a width W_{F} between the first and second side supports 414a, 414b. The width W_{F} is greater than the thickness T_{F} of the body 332 of the blade fitting 330 such that the seat 419 is configured to receive the body 332 of the blade fitting 330 therein.

The hinge pin 418 is attached to (i.e., extends through) the front of each of the first and second side supports 414a, 414b and extends across the seat 419. The hinge pin 418 is a cylinder having a diameter d_{F}. The diameter d_{F} is less than the diameter of the semicircular bearing surface 335 of the blade fitting 330 of the fiber-illuminated blade 300 such that the hinge pin 418 is configured to be received within, and be rotatably supported by, the hinge slot 334. The hinge lock 336 is configured to lock the hinge pin 418 within the hinge slot 334.

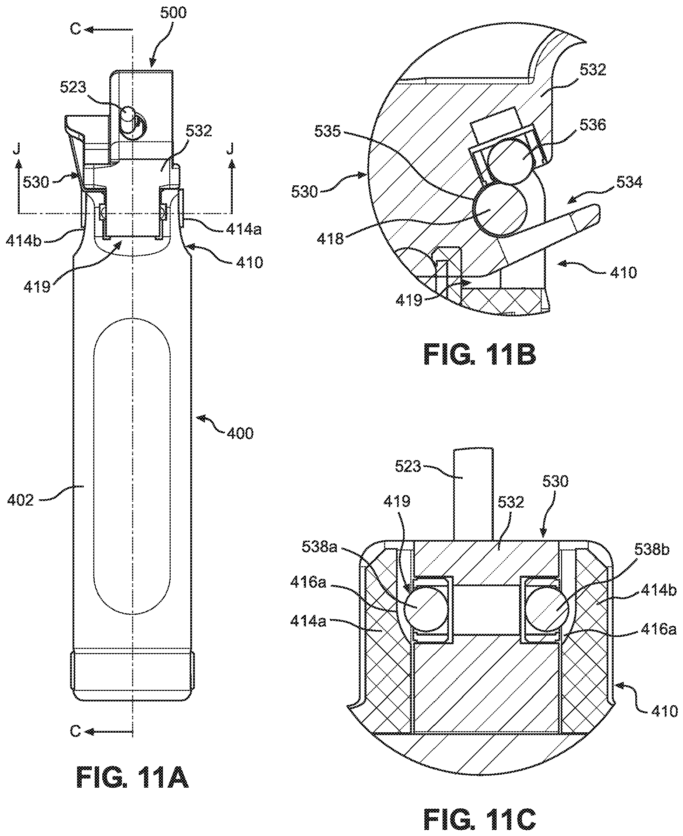

The first and second side supports 414a, 414b respectively include first and second lock slots 416a, 416b that protrude into a seat-facing side of the respective side support (the second lock slot 416b is shown in FIG. 11C, discussed below). The first and second lock slots 416a, 416b are configured to receive a respective one of the first and second side locks 338a, 338b of the blade fitting 330 to hold the body 332 within the seat 419. The first and second lock slots 416a, 416b are each respectively positioned with respect to a center point of the hinge pin 418 to ensure that the first and second side locks 338a, 338b fit within respective ones of the first and second lock slots 416a, 416b when the fiber-illuminated blade 300 is engaged with the fiber-illuminated handle 400 in the operating position. As shown in FIG. 4C, a center of the first lock slot 416a is spaced from the center point of the hinge pin 418, designated at the intersection of the hinge pin crosshairs, by a distance having a horizontal distance component D_{FLS_h}, and a vertical distance component D_{FLS_v}. The center of the first lock slot 416a is spaced rearwardly from the center point of the hinge pin 418 by the horizontal distance component D_{FLS_h}, and is spaced below the center point of the hinge pin 418 by the vertical distance component D_{FLS_v}. A center point of the second lock slot 416b is provided at a corresponding position at the seat-facing side of the second side support 414b with respect to the center point of the hinge pin 418 such that the first and second lock slots 416a, 416b are spaced the same distance from the center of the hinge pin 418.

The blade fitting 330 of the fiber-illuminated blade 300 is configured to engage with hook-on fitting 410 of the fiber-illuminated handle 400 to lock the fiber-illuminated blade 300 within the fiber-illuminated handle 400 in an operating position, in which the laryngoscope is ready-for-use. To place the fiber-illuminated blade 300 in the operating position, the blade fitting 330 is first attached to the hook-on fitting 410. The blade fitting 330 is attached to the hook-on fitting 410 of the fiber-illuminated handle 400 by placing the hinge pin 418 of the hook-on fitting 410 within the hinge slot 334 of the blade fitting 330 and providing relative movement between the hinge pin 418 and the hinge slot 334 until the hinge pin 418 abuts against the semicircular bearing surface 335 of the hinge slot 334 and is locked therein by the hinge lock 336. Relative rotational movement is provided between the fiber-illuminated blade 300 and the fiber-illuminated handle 400 such that the hinge slot 334 and the hinge pin 418 are rotated relative to one and other until the body 332 of the blade fitting 330 slides within the seat 419 of the hook-on fitting 410. When the body 332 of the blade fitting 330 is fully received within the seat 419 of the hook-on fitting 410, the first and second side locks 338a, 338b of the blade fitting 330 respectively engage with the first and second lock slots 416a, 416b of the hook-on fitting 410. The fiber-illuminated blade 300 is thus locked within the fiber-illuminated handle 400 in the operating position. In the operating position, the light guide interface 339 of the blade fitting 330 is engaged with the optical pathway 412 of the hook-on fitting 410, and the laryngoscope is ready-for-use.

Critical dimensions of conventional blades, conventional handles, fiber-illuminated blades, and fiber-illuminated handles conform to International Organization for Standardization (i.e., ISO) - International Standard 7376 for "Anesthetic and respiratory equipment - Laryngoscopes for tracheal intubation," which provides general requirements for laryngoscopes used for intubation. Because of the fitting standardization provided by ISO Standard 7376, conventional blades of varying types and sizes are interchangeably connectable with conventional handles of varying sizes and shapes. Similarly, fiber-illuminated blades of varying types and sizes are interchangeably connectable with fiber-illuminated handles of varying types and sizes.

With known interchangeable laryngoscope blades and handles, conventional blades will not function with fiber-illuminated handles, since fiber-illuminated handles do not include electrical contacts to supply the lamp of the conventional blade with power from batteries disposed within the handle. Similarly, fiber-illuminated blades will not function with conventional handles, since conventional handles include neither the lamp nor the optical pathway for interfacing with the light guide of the fiber-illuminated blade.

Accordingly, the dimensions of the blade fittings and hook-on fittings prescribed by ISO Standard 7376 intentionally limits the mechanical interchangeability of laryngoscope blades and handles to prevent handle/blade engagement that will not properly function (e.g., that will not produce light to illuminate the larynx). That is, blade fittings of conventional blades dimensioned to ISO Standard 7376 will not engage with hook-on fittings of fiber-illuminated handles dimensioned to ISO Standard 7376 for fiber-illuminated handles, and blade fittings of fiber-illuminated blades dimensioned to ISO Standard 7376 will not engage with hook-on fittings of conventional handles dimensioned to ISO Standard 7376 for conventional handles. For example, the thickness T_{C} of the body 132 of the conventional blade 100 is less than the thickness T_{F} of the body 332 of the fiber-illuminated blade 300, and the width W_{C} of the seat 219 of the conventional handle 200 is less than the width W_{F} of the seat 419 of the fiber-illuminated handle 400. Due to this configuration, engagement between the blade fitting 330 of the fiber-illuminated blade 300 and the hook-on fitting 210 of the conventional handle 200 is prevented because the thickness T_{F} of the body 332 of the fiber-illuminated blade 300 is thicker than the width W_{C} of the seat 219 of the conventional handle 200. That is, the body 332 of the fiber-illuminated blade 300 is too thick to fit within the seat 219 of the conventional handle 200.

Further, the diameter of the semicircular bearing surface 135 of the hinge slot 134 of the conventional blade 100 is smaller than the diameter of the semicircular bearing surface 335 of the hinge slot 334 of the fiber-illuminated blade 300, and the diameter d_{C} of the hinge pin 218 of the conventional handle 200 is smaller than the diameter d_{F} of the hinge pin 418 of the fiber-illuminated handle 400. Due to this configuration, engagement between the blade fitting 130 of the conventional blade 100 and the hook-on fitting 410 of the fiber-illuminated handle 400 is prevented because the diameter d_{F} of the hinge pin 418 of the fiber-illuminated handle 400 is too large to fit properly within the semicircular bearing surface 135 of the hinge slot 134 of the conventional blade 100.

The limited interchangeability of laryngoscope blades and handles renders obsolete otherwise useful laryngoscope handles that do not interface with incompatible laryngoscope blades. The limited interchangeability can limit a users' choice of laryngoscope blades. For example, if an institution (e.g., a hospital) makes a significant investment in fiber-illuminated laryngoscope blades and handles, it is costly to later switch to conventional blades because doing so would render obsolete the stock of otherwise useful fiber-illuminated handles that are not connectable with the conventional blades.
GB2491189 discloses a laryngoscope blade with built-in light and power sources. US7,824,331 discloses a laryngoscope blade in conformity with ISO-7376 including conductive connections for powering a light source.

### Summary

The invention is defined by the independent claim.

A selection of optional features is set out in the dependent claims.

### Brief Description of Drawings

In order that the invention may be readily understood, aspects of the invention are illustrated by way of examples in the accompanying drawings; however, the subject matter is not limited to the disclosed aspects.
FIG. 1A shows a side view of a known conventional blade.
FIG. 1B shows a rear view of the known convention blade.
FIG. 1C shows a magnified side view of the blade fitting of the known conventional blade.
FIG. 2A shows a perspective view of a known conventional handle.
FIG. 2B shows a top view of a hook-on fitting of the known conventional handle.
FIG. 2C shows a cut-away side view of the hook-on fitting of the known conventional handle taken along section B-B of FIG. 2B.
Fig. 3A shows a side view of a known fiber-illuminated blade.
FIG. 3B shows a rear view of the known fiber-illuminated blade.
FIG. 3C shows a magnified side view of the blade fitting of the known fiber-illuminated blade.
FIG. 4A shows a perspective view of a known fiber-illuminated handle.
FIG. 4B shows a top view of the hook-on fitting of the known fiber-illuminated handle.
FIG. 4C shows a cut-away side view of the hook-on fitting of the known fiber-illuminated handle taken along section A-A of FIG. 4B.
FIG. 5A shows a side view of an exemplary universal laryngoscope blade having at least one cable for direct connection to an external device in accordance with aspects of the invention.
FIG. 5B shows a schematic representation of an exemplary embodiment of a viewer of the universal laryngoscope and of an external device in accordance with aspects of the invention.
FIG. 6A shows a side view of another exemplary universal laryngoscope blade embodiment having a viewer without any direct connection to an external device in accordance with aspects of the invention.
FIG. 6B shows a schematic representation of another exemplary embodiment of the viewer of the universal laryngoscope and of the external device in accordance with aspects of the invention.
FIG. 7A shows a magnified rear view of a blade fitting in accordance with aspects of the invention.
FIG. 7B shows a magnified side view of the blade fitting in accordance with aspects of the invention.
FIG. 8A shows a side view of the universal laryngoscope blade hooked-on to the hinge pin of the conventional handle in accordance with aspects of the invention.
FIG. 8B shows a side view of the universal laryngoscope blade engaged with the conventional handle in the operating position in accordance with aspects of the invention.
FIG. 9A shows a rear view of the universal laryngoscope blade engaged with the conventional handle in the operating position in accordance with aspects of the invention.
FIG. 9B shows a magnified cross-sectional view of the engagement between blade fitting of the universal laryngoscope blade and the hook-on fitting of the conventional handle of FIG. 9A taken along section F-F.
FIG. 9C shows a magnified cross-sectional view of the engagement between the blade fitting of the universal laryngoscope blade and the hook-on fitting of the conventional handle of FIG. 9A taken along section H-H.
FIG. 10A shows a side view of the universal laryngoscope blade hooked-on to the hinge pin of the fiber-illuminated handle in accordance with aspects of the invention.
FIG. 10B shows a side view of the universal laryngoscope blade engaged with the fiber-illuminated handle in the operating position in accordance with aspects of the invention.
FIG. 11A shows a rear view of the universal laryngoscope blade engaged with the fiber-illuminated handle in the operating position in accordance with aspects of the invention.
FIG. 11B shows a magnified cross-sectional view of the engagement between blade fitting of the universal laryngoscope blade and the hook-on fitting of the fiber-illuminated handle of FIG. 11A taken along section C-C.
FIG. 11C shows a magnified cross-sectional view of the engagement between the blade fitting of the universal laryngoscope blade and the hook-on fitting of the fiber-illuminated handle of FIG. 11A taken along section J-J.
FIG. 12A shows another implementation of a universal laryngoscope blade engaged to a conventional handle in an operating position.
FIG. 12B shows a magnified view of FIG. 12A depicting the blade fitting of the universal laryngoscope blade engaged to a hook-on fitting of the conventional handle in an operating position.
FIG. 13 shows a process of using universal laryngoscope blades in accordance with aspects of the invention.

Features of the universal laryngoscope blade and associated methods according to aspects of the invention are described with reference to the drawings, in which like reference numerals refer to like parts throughout.

### Detailed Description

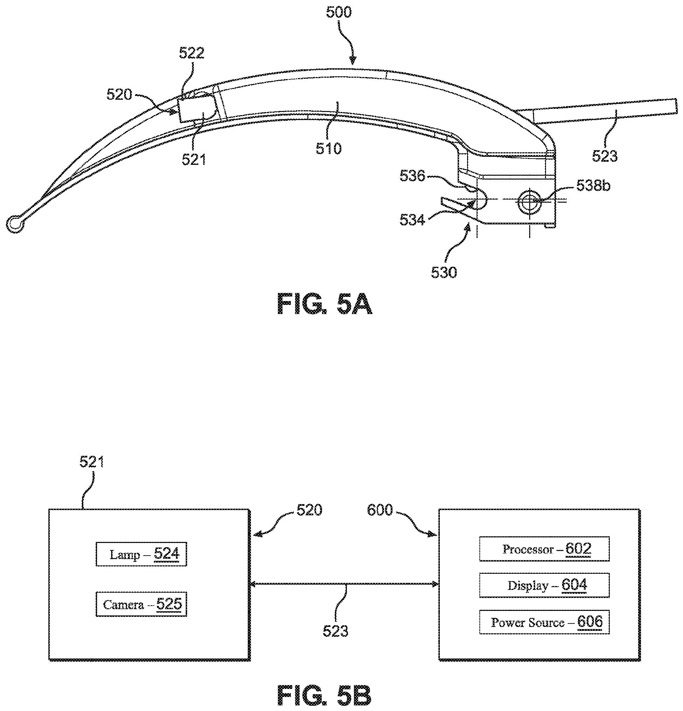

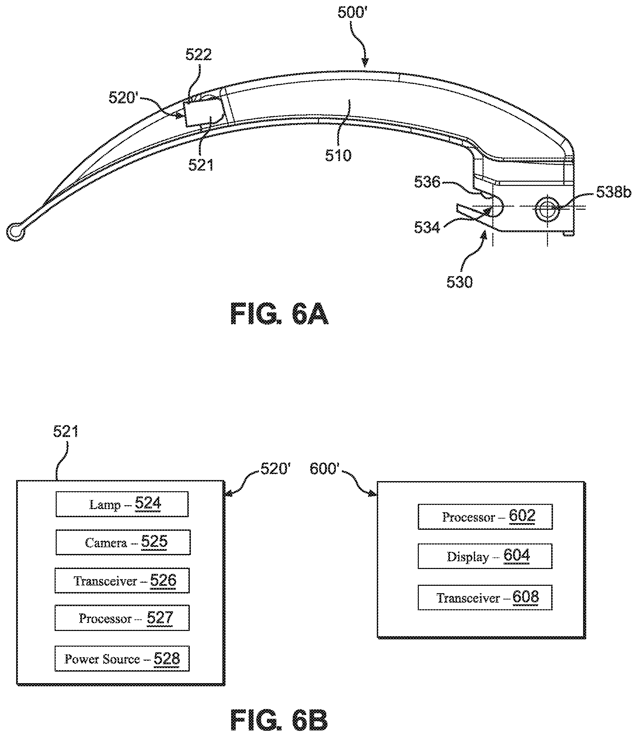

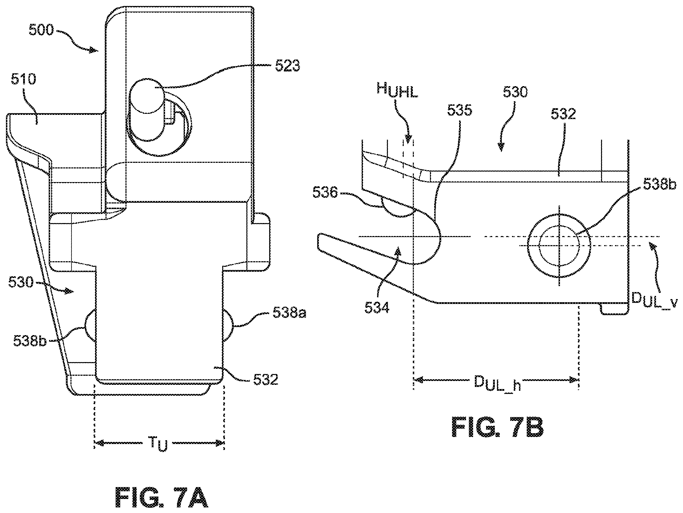

FIGS. 5A-7B show exemplary views of universal laryngoscope blades in accordance with aspects of the invention. FIG. 5A shows a side view of an exemplary universal laryngoscope blade 500 having at least one cable 523 for direct connection to an external device 600. FIG. 5B shows a schematic representation of an exemplary embodiment of the viewer 520 and the external device 600. FIG. 6A shows a side view of another exemplary universal laryngoscope blade 500' having a viewer 520' without any direct connection to an external device 600'. FIG. 6B shows a schematic representation of another exemplary embodiment of the viewer 520' and the external device 600'. FIG. 7A shows a magnified rear view of the blade fitting 530 in accordance with aspects of the invention. FIG. 7B shows a magnified side view of the blade fitting 530 in accordance with aspects of the invention.

The universal laryngoscope blade 500 includes a blade body 510, a viewer 520, and a blade fitting 530. The universal laryngoscope blade 500 may be, entirely or partially, reusable or disposable. The blade body 510 may be shaped to provide a direct view of a larynx. The blade body 510 may be provided in any number of shapes and sizes depending, e.g., on the size of the patient, as would be readily understood by a person having ordinary skill in the art. The blade body 510 may include a first end and a second end. The second end may be inserted into the larynx of a patient.

The viewer 520 is connected to the blade body 510 and may illuminate the larynx when the second end of the blade body 510 is inserted therein. The viewer 520 may function (e.g., emit light, capture at least one image, etc.) independently from each of the conventional handle 200 and the fiber-illuminated handle 400. That is, the viewer 520 does not rely on a laryngoscope handle for power, signal transmission, light emission, etc. The viewer 520 may include a housing 521. The housing 521 may be provided within a chamber of the blade body 510. Additionally, or alternatively, the housing 521 may be attached to an outer side of the blade body 510. The housing 521 may include an opening 522, positioned between the second end of the blade body 510 and the central portion of the blade body 510. The opening 522 may open towards the second end of the blade body 510. The opening 522 may be covered with a transparent window to seal an interior of the housing 521. The transparent window may include a lens. The viewer 520 may include one or more cables 523. The one or more cables 523 may define the housing 521. The one or more cables 523 may extend from a rear of the blade body 510 for connection to an external device 600. The external device 600 may be a device other than a laryngoscope handle. The external device 600 may include one or more of, e.g., a processor 602, a display 604, a power source 606, a lamp, a transceiver 608 (the term "transceiver" as used herein includes one or more of each of a transmitter and a receiver combined into a single circuitry, or provided separately, capable of transmitting and receiving electronic signals), etc. The one or more cables 523 may be capable of transmitting light, electricity, data, etc., between the viewer 520 and the external device 600.

The viewer 520 may include a lamp 524 that emits light from the opening 522of the housing 521 in a direction towards the second end of the blade body 510 to illuminate the second end of the blade body 510 and/or the larynx. The lamp 524 may be provided within the housing 521. The lamp 524 may be hermetically sealed within the housing 521. Alternatively, the external device 600 may include the lamp 524, and light may be directed through the one or more cables 523 to the opening 522 to illuminate the second end of the blade body 510 and/or the larynx.

The viewer 520 may include a camera 525 that may capture one or more images, from the perspective of the opening 522 of the housing 521, of the second end of the blade body 510 and/or of the larynx. At least a portion of the camera 525, and in embodiments the entire camera 525, may be provided within the housing 521. The camera 525 may be hermetically sealed within the housing 521. Images collected by the camera 525 may be electronically transferred to the external device 600. For example, the images may be electronically transferred via the one or more cables 523. Additionally, or alternatively, the viewer 520 may include a transceiver 526 that may wirelessly transmit the images to the external device 600. The transceiver 526 may receive control instructions from the processor 602. The viewer 520 may also include a processor 527 that may control any of the lamp 524, camera 525, transceiver 526, etc. The processor 527 may independently control the lamp 524, camera 525, and/or transceiver 526, or may be used to implement control instructions transmitted from the external device 600 and received by the transceiver 526.

The viewer 520 may include a power source 528 (e.g., a battery) that supplies power to the lamp 524, camera 525, transceiver 526, processor 527, etc. The power source 528 may be provided on the blade body 510. The powers source 528 may be positioned within, or connected to, the housing 521. Additionally, or alternatively, power may be supplied to the lamp 524 and/or the camera 525 from the external device 600 via the one or more cables 523.

As shown in FIGS. 5A and 5B, according to an embodiment of the invention, the viewer 520 may include the one more cables 523 that may supply light, electricity, and/or data. The viewer 520 may also include the lamp 524, and optionally the camera 525. The external device 600 may include a processor 602, a display 604, and a power source 606. The one or more cables 523 provide electronic communication between, e.g., the external device 600, and the lamp 524 and the camera 525. For example, the one or more cables 523 transmit power to the lamp 524 and to the camera 525. Images captured by the camera 525 are transmitted to the external device 600 via the one or more cables 523. The processor 602 may control the lamp 524 and the camera 525 via signals sent through the at one or more cables 523.

As shown in FIGS. 6A and 6B, according to another embodiment of the invention, the viewer 520' may be provided without any direct connection (i.e., cable link) with the external device 600'. For example, the viewer 520' may include the lamp 524, and optionally the camera 525. The viewer 520' also may include a power source 528 (e.g., a battery), a transceiver 526, and a processor 527. The external device 600' may include a processor 602, a display 604, and a transceiver 608. According to aspects invention, the viewer 520' is fully functional (i.e., capable of producing/transmitting light/data, capable of capturing one or more images, etc.) without the need for the blade fitting 530 to interface with functional elements (e.g., the electrical contact 212 of the conventional handle 200 or the optical pathway 412 of the fiber-illuminated handle 400) on the conventional handle 200 or the fiber-illuminated handle 400. In other words, the viewer 520' is self-contained in that it may draw power form a source (e.g., an internal power source 528) other than the handle.

The blade fitting 530 is disposed at the first end of the blade body 510. As discussed above, the hook-on fitting 210 of the conventional handle 200 has at least one physical dimension (e.g., the diameter d_{C} of the hinge pin 218, the width W_{C} of the seat 219, etc.) that is different from at least one physical dimension (e.g., the diameter d_{F} of the hinge pin 418, the width W_{F} of the seat 419) of the hook on fitting of the fiber-illuminated blade 300. Further, the hook-on fitting 210 of the conventional handle 200 is dimensioned to conform to ISO standard 7376 for conventional handles and the hook-on fitting 410 of the fiber-illuminated handle 400 is dimensioned to conform to ISO standard 7376 for fiber-illuminated handles. Nevertheless, the blade fitting 530 of the present invention is removably connectable to, and functional with, each of the hook-on fitting 210 of the conventional handle 200 and the hook-on fitting 410 of the fiber-illuminated handle 400. The blade fitting 530 of the invention may also be adapted for removable connection to, and functionality with, hook-on fittings of other handles that differ from the hook-on fitting 210 of the conventional handle 200 and the hook-on fitting 410 of the fiber-illuminated handle 400 described herein.

The blade fitting 530 of the universal laryngoscope blade 500 includes a body 532, a lock, and a hinge slot 534. As shown in FIG. 7A, the body 532 has a thickness Tu. The thickness Tu of the body 532 of the universal laryngoscope blade 500 may be greater than the thickness Tc of the body of the conventional blade 100 but less than the thickness T_{F} of the body 332 of the fiber-illuminated blade 300. For example, the thickness Tu may be between 12.74 and 12.84 mm, and more particularly may be 12.80 mm. According to aspects of the invention, because the thickness Tu of the body 532 of the universal laryngoscope blade 500 may be greater than the thickness Tc of the body 132 of the conventional blade 100 but less than the thickness T_{F} of the body 332 of the fiber-illuminated blade 300, the body 532 of the universal laryngoscope blade 500 may be small enough to be received within the seat 219 of the conventional handle 200 while large enough to limit excessive play (wobbling) when received within the seat 419 of the fiber-illuminated handle 400. That is, the body 532 of the universal laryngoscope blade 500 may be accommodated within, one at a time, the seat 219 of the conventional handle 200 and the fiber-illuminated handle 400, improving the universality of blade fitting 530 of the universal laryngoscope blade 500. A bottom of the body 532 may not include (i.e., may be free of) functional elements (e.g., electrical contacts, light guides, etc.) that would otherwise protrude from the body 532. By not including functional elements on the body 532 of the blade fitting 530, the bottom of the body 532 of the blade fitting 530 may not interfere with functional elements (e.g., electrical contacts, optical pathways, etc.) provided on the hook-on fittings of conventional handles and/or fiber-illuminated handles when connected thereto. Alternatively, the bottom of the body 532 may be equipped with a switch that activates the viewer 520 when the universal laryngoscope blade 500 is engaged with the conventional handle 200/the fiber-illuminated handle 400 in the operating position.

The lock may include a hinge lock 536 disposed within a top surface of the hinge slot 534. In embodiments, the hinge lock 536 may be disposed within a bottom surface of the hinge slot 534. The lock may also include at least one side lock provided on at least one side of the body 532. As is shown in FIG. 7A, the at least one side lock may include first and second side locks 538a, 538b respectively provided on opposite sides of the body 532. A center point of the second side lock 538b is indicted at the intersection of side lock crosshairs, as shown in FIG. 7B. The hinge lock 536, the first side lock 538a, and/or the second side lock 538b may be detent mechanisms. The hinge slot 534 may extend into the body 532 at an angle from a front of the body 532 towards a central region of the body 532 and may terminate at a bearing surface 535. The bearing surface 535 has semicircular shape having a diameter and a virtual center point, indicated by the intersection of hinge slot 534 crosshairs shown in FIG. 7B, about which the bearing surface 535 forms the semicircular shape. The diameter of the semicircular bearing surface 535 of the hinge slot 534, and a width of the hinge slot 534 in general, are dimensioned to accommodate, one at a time, the hinge pin 218 of the conventional handle 200 and the hinge pin 418 of the fiber-illuminated handle 400 such that the respective hinge pin is received within and abuts against the semicircular bearing surface 535. That is, the hinge slot 534 is dimensioned to receive in abutment with the semicircular bearing surface 535, one at a time, the hinge pin 218 of the hook-on fitting 210 of the conventional handle 200 and the hinge pin 418 of hook-on fitting 410 of the fiber-illuminated handle 400. For example, the diameter of the semicircular bearing surface 535 of the hinge slot 534, and a narrowest width of the hinge slot 534 in general, are each at least greater than 4.58 mm.

As shown in FIG. 7B, a center of the hinge lock 536 is spaced forwardly from the virtual center of the semicircular bearing surface 535 by a horizontal distance H_{UHL}. The horizontal distance H_{UHL} that the hinge lock 536 of the universal laryngoscope blade 500 is spaced forwardly from the virtual center of the semicircular bearing surface 535 of the universal laryngoscope blade 500 is less than the horizontal distance H_{FHL} that the hinge lock 336 of the fiber-illuminated blade 300 is spaced forwardly from the virtual center of the semicircular bearing surface 335 of the fiber-illuminated blade 300. For example, the horizontal distance H_{UHL} that the hinge lock 536 of the universal laryngoscope blade 500 is spaced forwardly from the virtual center of the semicircular bearing surface 535 of the universal laryngoscope blade 500 may be between 0.86 and 0.90 mm, and more particularly may be 0.88 mm. By locating the hinge lock 536 the horizontal distance H_{UHL} forwardly from the virtual center of the semicircular bearing surface 535, the hinge lock 536 may securely hold, one at a time, the hinge pin 218 of the conventional handle 200 and the hinge pin 418 of the fiber-illuminated handle 400 within the hinge slot 534 of the blade fitting 530. That is, the hinge slot 534 and the hinge lock 536 of the universal laryngoscope blade 500 may accommodate, one at a time, the hinge pin 218 of the conventional handle 200, and the larger hinge pin 418 of the fiber-illuminated handle 400, improving the universality of the blade fitting 530 of the universal laryngoscope blade 500.

The center point of the second side lock 538b may be spaced from the virtual center point of the semicircular bearing surface 535 by a distance having a horizontal distance component D_{UL_h}, and a vertical distance component D_{UL_v}. The center point of the second side lock 538b may be spaced rearwardly from the virtual center point of the semicircular bearing surface 535 by the horizontal distance component D_{UL_h}, and may be spaced below the virtual center point of the bearing surface 535 by the vertical distance component D_{UL_v}. A center point of the first side lock 538a may be provided at a corresponding position at the other side surface of the body 532 with respect to the virtual center point of the semicircular bearing surface 535 such that the first and second side locks 538a, 538b are spaced the same distance from the virtual center point of the semicircular bearing surface 535.

The horizontal distance component D_{UL_h}, and the vertical distance component D_{UL_v} may be set such that the side lock (i.e., the first side lock 538a or the second side lock 538b) may be accommodated within a respective lock slot of the conventional handle 200 when the universal laryngoscope blade 500 is engaged with the hook-on fitting 210 of the conventional handle 200 in an operating position, in which the laryngoscope is ready-for-use. The horizontal distance component D_{UL_h}, and the vertical distance component D_{UL_v} may be set such that the side lock (i.e., the first side lock 538a or the second side lock 538b) may also be accommodated within a respective lock slot of the fiber-illuminated handle 400 when the universal laryngoscope blade 500 is engaged with the hook-on fitting 410 of the fiber-illuminated handle 400 in the operating position. For example, the horizontal distance component D_{UL_h} may be of a magnitude greater than the horizontal distance component D_{CL_h} of the blade fitting 130 of the conventional blade 100 and less than the horizontal distance component D_{FLh} of the blade fitting 330 of the fiber-illuminated blade 300. The horizontal distance component D_{UL_h} may be 13.0 mm. The vertical distance component D_{UL_v} may be 0.8 mm.

As a result of the above-described features and dimensions (e.g., the thickness Tu of the body 532, the width of the hinge slot 534, the diameter of the semicircular bearing surface 535 of the hinge slot 534, the horizontal distance H_{UHL}, the horizontal distance component D_{UL_h}, the vertical distance component D_{UL_v}, etc.), the blade fitting 530 of the universal laryngoscope blade 500 may engage with, one at a time, both the hook-on fitting 210 of the conventional handle 200 and the hook-on fitting 410 of the fiber-illuminated handle 400. Further, and as discussed above, the viewer 520 (or the viewer 520') is fully functional (i.e., capable of producing/transmitting light/data, capable of capturing one or more images, etc.) without the need for the blade fitting 530 to interface with functional elements (e.g., the electrical contact 212 of the conventional handle 200 or the optical pathway 412 of the fiber-illuminated handle 400) on the conventional handle 200 or the fiber-illuminated handle 400. Accordingly, the universal laryngoscope blade 500 may be attached to, and fully functional with, each of the conventional handle 200 and the fiber-illuminated handle 400 that are dimensioned to conform to the requirements of ISO Standard 7376. This may allow a user to remove handle compatibility from laryngoscope blade purchasing decisions to leverage initial investments in otherwise useful conventional handles and/or fiber-illuminated handles. Universal laryngoscope blades may also reap the benefits of economies of scale, since the universal laryngoscope blades may be produced in greater numbers due to the compatibility with both existing conventional handles and fiber-illuminated handles.

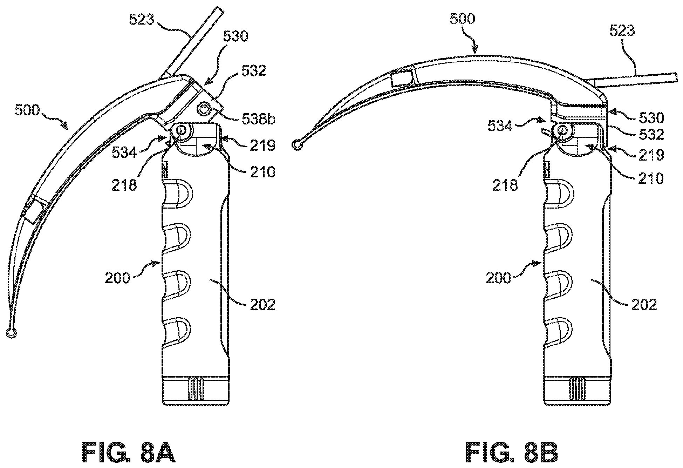

FIGS. 8A-9C depict engagement of the universal laryngoscope blade 500 and the conventional handle 200. FIG. 8A shows a side view of the universal laryngoscope blade 500 hooked-on to the hinge pin 218 of the conventional handle 200. FIG. 8B shows a side view of the universal laryngoscope blade 500 engaged with the conventional handle 200 in the operating position. FIG. 9A shows a rear view of the universal laryngoscope blade 500 engaged with the conventional handle 200 in the operating position. FIG. 9B shows a magnified cross-sectional view of the engagement between blade fitting 530 of the universal laryngoscope blade 500 and the hook-on fitting 210 of the conventional handle 200 of FIG. 9A taken along section F-F. FIG. 9C shows a magnified cross-sectional view of the engagement between the blade fitting 530 of the universal laryngoscope blade 500 and the hook-on fitting 210 of the conventional handle 200 of FIG. 9A taken along section H-H.

The blade fitting 530 of the universal laryngoscope blade 500 is configured to engage with the hook-on fitting 210 of the conventional handle 200 to lock the universal laryngoscope blade 500 within the seat 219 of the conventional handle 200 in an operating position, in which the laryngoscope is ready-for-use. As shown in FIG. 8A, to place the universal laryngoscope blade 500 in the operating position, the blade fitting 530 is first attached to the hook-on fitting 210. The blade fitting 530 is attached to the hook-on fitting 210 of the conventional handle 200 by placing the hinge pin 218 of the hook-on fitting 210 within the hinge slot 534 of the blade fitting 530 and providing relative movement between the hinge pin 218 and the hinge slot 534 until the hinge pin 218 abuts against the semicircular bearing surface 535 of the hinge slot 534 and is locked therein by the hinge lock 536. FIG. 9B shows the hinge lock 536 locking the hinge pin 218 of the conventional handle 200 within the hinge slot 534 of the universal laryngoscope blade 500 in the operating position. Relative rotational movement is provided between the universal laryngoscope blade 500 and the conventional handle 200 such that the hinge slot 534 and the hinge pin 218 are rotated relative to one and other until the body 532 of the blade fitting 530 slides within the seat 219 of the hook-on fitting 210, as shown in FIGS. 8B-9C. After the body 532 of the blade fitting 530 is fully received within the seat 219 of the hook-on fitting 210, the first and second side locks 538a, 538b of the blade fitting 530 respectively engage with the first and second lock slots 216a, 216b of the hook-on fitting 210, as shown in FIG. 9C. With the side locks engaged with the respective lock slots and the hinge lock 536 engaged with the hinge pin 218, the body 532 of the universal laryngoscope blade 500 is locked within the seat 219 of the conventional handle 200 in the operating position, and the laryngoscope is ready-for-use. The one or more cables 523 of the universal laryngoscope blade 500 may be connected to the external device 600 at any time prior to use.

Although FIGS. 8A-9C depict engagement between the universal laryngoscope blade 500 having one or more cables 523 and the conventional handle 200, the invention also includes engagement between the universal laryngoscope blade 500 having the viewer 520' without any direct connection (i.e., cable link) with the external device 600 and the conventional handle 200. That is, the mechanical engagement between the blade fitting 530 of the universal laryngoscope blade 500 and the hook-on fitting 210 of the conventional handle 200 is the same regardless of the type of viewer employed by the universal laryngoscope blade 500. Each universal laryngoscope blade 500 is self-contained in that the universal laryngoscope blade 500 does not require power or light from the conventional handle 200 (although in some embodiments power or light may be provided by an external device 600 other than the conventional handle 200).

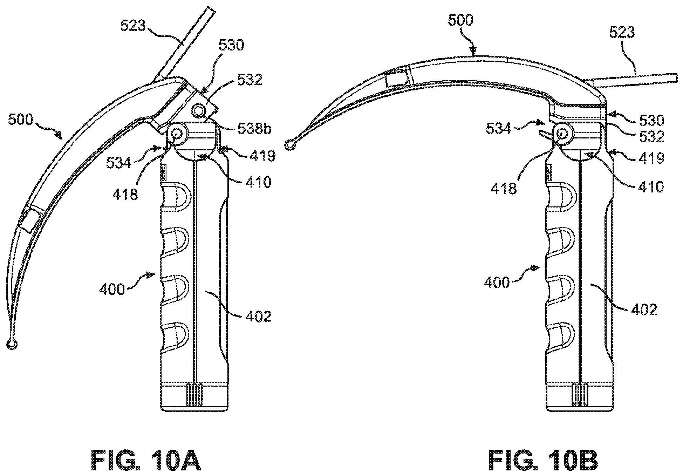

FIGS. 10A-11C depict engagement of the universal laryngoscope blade 500 and the fiber-illuminated handle 400. FIG. 10A shows a side view of the universal laryngoscope blade 500 hooked-on to the hinge pin 418 of the fiber-illuminated handle 400. FIG. 10B shows a side view of the universal laryngoscope blade 500 engaged with the fiber-illuminated handle 400 in the operating position. FIG. 11A shows a rear view of the universal laryngoscope blade 500 engaged with the fiber-illuminated handle 400 in the operating position. FIG. 11B shows a magnified cross-sectional view of the engagement between blade fitting 530 of the universal laryngoscope blade 500 and the hook-on fitting 410 of the fiber-illuminated handle 400 of FIG. 11A taken along section C-C. FIG. 11C shows a magnified cross-sectional view of the engagement between the blade fitting 530 of the universal laryngoscope blade 500 and the hook-on fitting 410 of the fiber-illuminated handle 400 of FIG. 11A taken along section J-J.

The blade fitting 530 of the universal laryngoscope blade 500 is also configured to engage with the hook-on fitting 410 of the fiber-illuminated handle 400 to lock the universal laryngoscope blade 500 within the seat 419 of the fiber-illuminated handle 400 in an operating position, in which the laryngoscope is ready-for-use. As shown in FIG. 10A, to place the universal laryngoscope blade 500 in the operating position, the blade fitting 530 is first attached to the hook-on fitting 410. The blade fitting 530 is attached to the hook-on fitting 410 of the fiber-illuminated handle 400 by placing the hinge pin 418 of the hook-on fitting 410 within the hinge slot 534 of the blade fitting 530 and providing relative movement between the hinge pin 418 and the hinge slot 534 until the hinge pin 418 abuts against the semicircular bearing surface 535 of the hinge slot 534 and is locked therein by the hinge lock 536. FIG. 11B shows the hinge lock 536 locking the hinge pin 418 of the fiber-illuminated handle 400 within the hinge slot 534 of the universal laryngoscope blade 500 in the operating position. Relative rotational movement is provided between the universal laryngoscope blade 500 and the fiber-illuminated handle 400 such that the hinge slot 534 and the hinge pin 418 are rotated relative to one and other until the body 532 of the blade fitting 530 slides within the seat 419 of the hook-on fitting 410, as shown in FIGS. 10B-11C. After the body 532 of the blade fitting 530 is fully received within the seat 419 of the hook-on fitting 410, the first and second side locks 538a, 538b of the blade fitting 530 respectively engage with the first and second lock slots 416a, 416b of the hook-on fitting 410, as shown in FIG. 11C. With the side locks engaged with the respective lock slots and the hinge lock 536 engaged with the hinge pin 418, the body 532 of the universal laryngoscope blade 500 is locked within the seat 419 of the fiber-illuminated handle 400 in the operating position, and the laryngoscope is ready-for-use. The one or more cables 523 of the universal laryngoscope blade 500 may be connected to the external device 600 at any time prior to use.

Although FIGS. 10A-11C depict engagement between the universal laryngoscope blade 500 having one or more cables 523 and the fiber-illuminated handle 400, the invention also includes engagement between the universal laryngoscope blade 500 having the viewer 520' without any direct connection (i.e., cable link) with the external device 600 and the fiber-illuminated handle 400. That is, the mechanical engagement between the blade fitting 530 of the universal laryngoscope blade 500 and the hook-on fitting 410 of the fiber-illuminated handle 400 is the same regardless of the type of viewer employed by the universal laryngoscope blade 500. Each universal laryngoscope blade 500 is self-contained in that the blade does not require power or light from the fiber-illuminated handle 400 (although in some embodiments power or light may be provided by an external device 600 other than the fiber-illuminated handle 400).

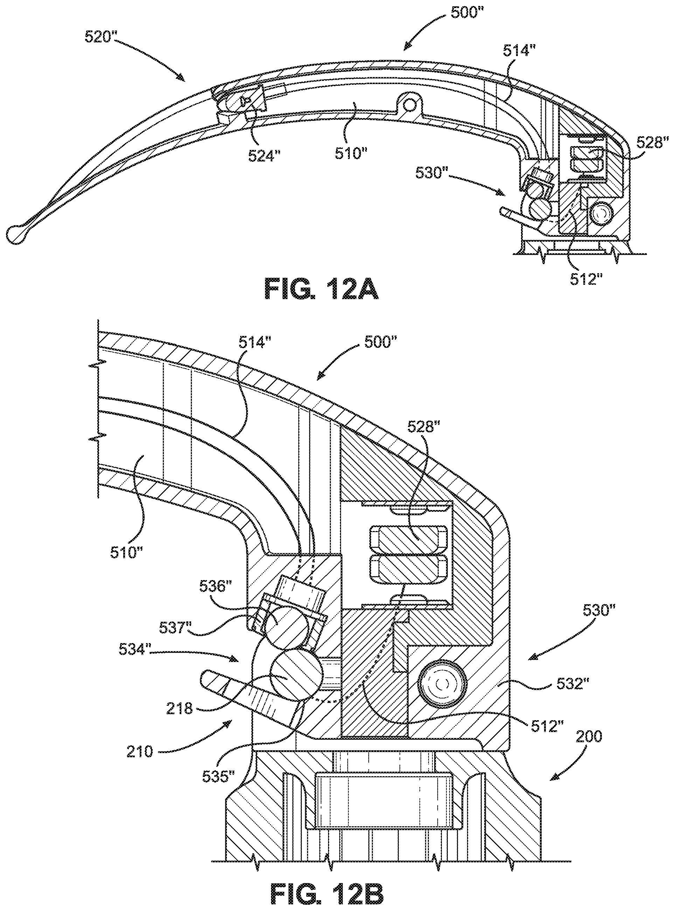

FIGS. 12A-12B show exemplary views of another implementation of a universal laryngoscope blade 500" in accordance with aspects of the present disclosure. The universal laryngoscope blade 500" includes a blade body 510", a viewer 520", a power source 528", and a blade fitting 530". The universal laryngoscope blade 500" may be, entirely or partially, reusable or disposable. The blade body 510" may be the same as the blade body 510 previously discussed in detail above with regards to the other universal laryngoscope blade implementations. For instance, the blade body 510" may be shaped to provide a direct view of a larynx. The blade body 510" may be provided in any number of shapes and sizes depending, e.g., on the size of the patient. The blade body 510" may include a first end and a second end, the power source 528" disposed at the first end, and the second end operable for insertion into the larynx of a patient.

For illustrations purposes, FIG. 12A depicts engagement of the universal laryngoscope blade 500" with the conventional handle 200. In particular, a side view of the universal laryngoscope blade 500" hooked-on to the hinge pin 218 of the conventional handle 200 is illustrated, such that the universal laryngoscope blade 500" is engaged with the conventional handle 200 in the operating position. FIG. 12B shows a magnified view of the engagement between blade fitting 530" of the universal laryngoscope blade 500" and the hook-on fitting 210 of the conventional handle 200.

The viewer 520" is connected to the blade body 510" and is operable to illuminate the larynx when the second end of the blade body 510" is inserted therein. The viewer 520" is operable to function (e.g., emit light, capture at least one image, etc.) independently with each of the conventional handle 200 and the fiber-illuminated handle 400. The viewer 520" may include a lamp 524", such as an LED, that emits light in a direction towards the second end of the blade body 510" to illuminate the second end of the blade body 510" and/or the larynx. Similar to the other implementations discussed above, the lamp 524" may be provided within a housing. The viewer 520" may include a camera that may capture one or more images, from the perspective of an opening of the housing, of the second end of the blade body 510" and/or of the larynx. The viewer 520" may include a power source 528" (e.g., one or more batteries) that supplies power to the lamp 524", camera, etc. The power source 528" may be provided on the blade body 510", such as the first end of the blade body.

The universal laryngoscope blade 500" is configured to be removably connectable, one at a time, to each of the conventional handle 200 and the fiber-illuminated handle 400. The blade fitting 530" of the laryngoscope blade 500" is disposed at the first end of the blade body 510". The blade fitting 530" is configured to be removably connectable, one at a time, to each of the hook-on fitting 210 of the conventional handle 200 and the hook-on fitting 410 of the fiber-illuminated handle 400. Moreover, the blade fitting 530" is removably connectable to and functional with, one at a time, each of the hook-on fitting 210 of the conventional handle 200 and the hook-on fitting 410 of the fiber-illuminated handle 400. In other implementations, the blade fitting 530" may also be adapted for removable connection to, and functionality with, hook-on fittings of other handles that differ from the hook-on fitting 210 of the conventional handle 200 and the hook-on fitting 410 of the fiber-illuminated handle 400 described herein.

The blade fitting 530" of the universal laryngoscope blade 500" includes a body 532", a lock, and a hinge slot 534". The body 532" is similar to the body 532 shown in FIG. 7A and has a thickness Tu. The thickness Tu of the body 532" of the universal laryngoscope blade 500" may be greater than the thickness Tc of the body of the conventional blade 100 but less than the thickness T_{F} of the body 332 of the fiber-illuminated blade 300. For example, the thickness Tu may be between 12.74 and 12.84 mm, and more particularly may be 12.80 mm. According to aspects of the invention, because the thickness Tu of the body 532" of the universal laryngoscope blade 500" may be greater than the thickness Tc of the body 132 of the conventional blade 100 but less than the thickness T_{F} of the body 332 of the fiber-illuminated blade 300, the body 532" of the universal laryngoscope blade 500" may be small enough to be received within the seat 219 of the conventional handle 200 while large enough to limit excessive play (wobbling) when received within the seat 419 of the fiber-illuminated handle 400. That is, the body 532" of the universal laryngoscope blade 500" may be accommodated within, one at a time, the seat 219 of the conventional handle 200 and the fiber-illuminated handle 400, improving the universality of blade fitting 530" of the universal laryngoscope blade 500".

The lock may include a hinge lock 536" disposed within a top surface of the hinge slot 534". In some implementations, the hinge lock 536" may be disposed within a bottom surface of the hinge slot 534". The lock may also include at least one side lock provided on at least one side of the body 532". The at least one side lock may include first and second side locks respectively provided on opposite sides of the body 532", similar to slide locks 538a, 538b previously described above. The hinge lock 536" and the at least one side lock may be detent mechanisms. The hinge slot 534" may extend into the body 532" at an angle from a front of the body 532" towards a central region of the body 532" and may terminate at a bearing surface 535". The bearing surface 535" may have a semicircular shape having a diameter and a virtual center point about which the bearing surface 535" forms the semicircular shape. The diameter of the semicircular bearing surface 535" of the hinge slot 534", and a width of the hinge slot 534" in general, are dimensioned to accommodate, one at a time, the hinge pin 218 of the conventional handle 200 and the hinge pin 418 of the fiber-illuminated handle 400 such that the respective hinge pin is received within and abuts against the semicircular bearing surface 535". That is, the hinge slot 534" is dimensioned to receive in abutment with the semicircular bearing surface 535", one at a time, the hinge pin 218 of the hook-on fitting 210 of the conventional handle 200 and the hinge pin 418 of hook-on fitting 410 of the fiber-illuminated handle 400. For example, the diameter of the semicircular bearing surface 535" of the hinge slot 534", and a narrowest width of the hinge slot 534" in general, are each at least greater than 4.58 mm.

A center of the hinge lock 536" is spaced forwardly from the virtual center of the semicircular bearing surface 535" by a horizontal distance H_{UHL}. The horizontal distance H_{UHL} that the hinge lock 536" of the universal laryngoscope blade 500" is spaced forwardly from the virtual center of the semicircular bearing surface 535" of the universal laryngoscope blade 500" is less than the horizontal distance H_{FHL} that the hinge lock 336 of the fiber-illuminated blade 300 is spaced forwardly from the virtual center of the semicircular bearing surface 335 of the fiber-illuminated blade 300. For example, the horizontal distance H_{UHL} that the hinge lock 536" of the universal laryngoscope blade 500" is spaced forwardly from the virtual center of the semicircular bearing surface 535" of the universal laryngoscope blade 500" may be between 0.86 and 0.90 mm, and more particularly may be 0.88 mm. By locating the hinge lock 536" the horizontal distance H_{UHL} forwardly from the virtual center of the semicircular bearing surface 535", the hinge lock 536" may securely hold, one at a time, the hinge pin 218 of the conventional handle 200 and the hinge pin 418 of the fiber-illuminated handle 400 within the hinge slot 534" of the blade fitting 530". That is, the hinge slot 534" and the hinge lock 536" of the universal laryngoscope blade 500" may accommodate, one at a time, the hinge pin 218 of the conventional handle 200, and the larger hinge pin 418 of the fiber-illuminated handle 400, improving the universality of the blade fitting 530" of the universal laryngoscope blade 500".

As a result of the above-described features and dimensions (e.g., the thickness Tu of the body 532", the width of the hinge slot 534", the diameter of the semicircular bearing surface 535" of the hinge slot 534", the horizontal distance H_{UHL}, etc.), the blade fitting 530" of the universal laryngoscope blade 500" may engage with, one at a time, both the hook-on fitting 210 of the conventional handle 200 and the hook-on fitting 410 of the fiber-illuminated handle 400. Accordingly, the universal laryngoscope blade 500" may be attached to, and fully functional with, each of the conventional handle 200 and the fiber-illuminated handle 400 that are dimensioned to conform to the requirements of ISO Standard 7376.

An electrical contact may be provided on the bearing surface 535" of the blade fitting 530". The electrical contact may be in electrical communication with the power source 528" (i.e., batteries) via one or more wires 512" for supplying power to the electrical contact. The electrical contact is further configured to be in electrical communication with the hinge pin 218 of the hook-on fitting 210 when the laryngoscope blade 500" is in the operating position. The hinge pin 218 is metal or another electrically conductive material. An electrical hinge contact 537" may be disposed on a surface of the hinge slot 534" and may be adjacent to the hinge lock 536". The electrical hinge contact 537" is also configured to be in electrical communication with the hinge pin 218 when the laryngoscope blade 500" is in the operating position. The electrical hinge contact 537" may further be in electrical communication with the viewer 520" via one or more wires 514" for supplying power to the viewer, such as to the lamp 524", when the laryngoscope blade 500" is engaged with the conventional handle 200 in the operating position.

Although the universal laryngoscope blade 500" is shown attached to and fully functional with the conventional handle 200 for illustrations purposes, the universal laryngoscope blade 500" is also attachable to and fully functional with the fiber-illuminated handle 400 as previously discussed above. Accordingly, power may be supplied from the power source 528" to the lamp 524" using the existing metal hinge pin 218 of the conventional handle 200 or the metal hinge pin 418 of the fiber-illuminated handle to complete the electrical circuit between the power source and the lamp, thus reducing manufacturing costs as it would be unnecessary to include additional components, such as a microswitch, into the blade.

In some implementations, the hinge lock 536" may be metal or another electrically conductive material, and furthermore may be configured to be in electrical communication with both the hinge pin 218 and the electrical hinge contact 537" when the laryngoscope blade 500" is in the operating position. Thus, a bottom of the body 532" may not include (i.e., may be free of) functional elements (e.g., electrical contacts, light guides, etc.) that would otherwise protrude from the body 532". By not including functional elements on the body 532" of the blade fitting 530", the bottom of the body 532" of the blade fitting 530" may not interfere with functional elements (e.g., electrical contacts, optical pathways, etc.) provided on the hook-on fittings of conventional handles and/or fiber-illuminated handles when connected thereto.

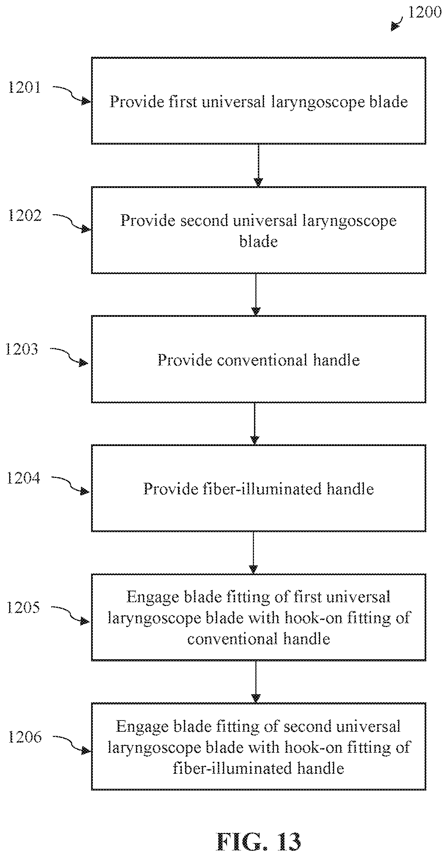

FIG. 13 shows a process 1200 of using universal laryngoscope blades in accordance with aspects of the invention, including engaging universal laryngoscope blades with a conventional handle and a fiber-illuminated handle (e.g., the conventional handle 200 and the fiber-illuminated handle 400). The process 1200 may include, at a first step 1201, providing a first universal laryngoscope blade. The first universal laryngoscope blade may be any of the universal laryngoscope blades discussed above in accordance with aspects of the invention. For example, the first universal laryngoscope blade may include a blade body shaped to provide a direct view of a larynx. The blade body may include a first end and a second end. The second end may be inserted into the larynx of a patient. The first universal laryngoscope blade may further include a viewer connected to the blade body. The viewer may function independently from each of the conventional handle and the fiber-illuminated handle. That is, the viewer is fully functional (i.e., able to emit light or capture images) without requiring power or light from the conventional handle or the fiber-illuminated handle. The first universal laryngoscope blade may further include a blade fitting disposed at the first end of the blade body.

The process 1200 may include, at a second step 1202, providing a second universal laryngoscope blade. The second universal laryngoscope blade may be any of the universal laryngoscope blades discussed above in accordance with aspects of the invention. For example, the second universal laryngoscope blade may include a blade body shaped to provide a direct view of a larynx. The blade body may include a first end and a second end. The second end may be inserted into the larynx of a patient. The second universal laryngoscope blade may further include a viewer connected to the blade body. The viewer may function independently from each of the conventional handle and the fiber-illuminated handle. That is, the viewer is fully functional (i.e., able to emit light or capture images) without requiring power or light from the conventional handle or the fiber-illuminated handle. The second universal laryngoscope blade may further include a blade fitting disposed at the first end of the blade body. the blade fitting of the second universal laryngoscope blade being identically dimensioned with the blade fitting of the first universal laryngoscope blade. That is, the physical dimensions of each of the above described components of the blade fitting of the universal laryngoscope blade (e.g., the body, the lock, the hinge slot, etc.) may be identical so that the blade fitting of each of the first and second universal laryngoscope blades is the same. "Identically dimensioned," as used herein, means machined to the same size, but includes normal variations resulting from manufacturing tolerances, which would be readily understood by persons having ordinary skill in the art.

The process 1200 may include, at a third step 1203, providing the conventional handle, as discussed above. That is, providing the conventional handle including a hook-on fitting having a seat having a width and a hinge pin having a diameter. The dimensions of the hook-on fitting of the conventional handle may conform to dimensions prescribed for conventional handles in ISO standard 7376.

The process 1200 may include, at a forth step 1204, providing the fiber-illuminated handle, as discussed above. That is, providing the fiber-illuminated handle including a hook-on fitting comprising a seat having a width and a hinge pin having a diameter. The dimensions of the hook-on fitting of the fiber-illuminated handle may conform to dimensions prescribed for fiber-illuminated handles for ISO standard 7376. As is clear from ISO standard 7376, the width of the seat of the fiber-illuminated handle is greater than the width of the seat of the conventional handle, and the diameter of the hinge pin of the fiber-illuminated handle is greater than the diameter of the hinge pin of the conventional handle.

The process 1200 may include, at a fifth step 1205, engaging the blade fitting of the first universal laryngoscope blade with the hook-on fitting of the conventional handle such that the first universal laryngoscope blade is provided in an operating position and ready-for-use. An example of engaging the blade fitting of the first universal laryngoscope blade with the hook-on fitting of the conventional handle is shown, for example, in FIGS. 8A-9C and is discussed in greater detail above.

The process 1200 may include, at a sixth step 1206, engaging the blade fitting of the second universal laryngoscope blade with the hook-on fitting of the fiber-illuminated handle such that the second universal laryngoscope blade is provided in an operating position and ready-for-use. An example of engaging the blade fitting of the second universal laryngoscope blade with the hook-on fitting of the fiber-illuminated handle is shown, for example, in FIGS. 10A-11C and is discussed in greater detail above.

Because the blade fittings of the first and second universal laryngoscope blades are identically dimensioned (including e.g., the thickness Tu of the body, the width of the hinge slot, the diameter of the semicircular bearing surface of the hinge slot, the horizontal distance H_{UHL}, the horizontal distance component D_{UL_h}, the vertical distance component D_{UL_v}, etc.), the blade fitting of the universal laryngoscope blades may each engage with, one at a time, both the hook-on fitting of the conventional handle and the hook-on fitting of the fiber-illuminated handle. Further, and as discussed above, the viewers of each of the universal laryngoscope blades are fully functional (i.e., capable of producing/transmitting light/data, capable of capturing one or more images, etc.) without the need for the blade fitting to interface with functional elements (e.g., the electrical contact of the conventional handle or the optical pathway of the fiber-illuminated handle) on the conventional handle or the fiber-illuminated handle. Accordingly, the universal laryngoscope blades may be attached to, and fully functional with, each of the conventional handle and the fiber-illuminated handle that conform to the requirements of ISO Standard 7376. This allows a user to remove handle compatibility from laryngoscope blade purchasing decisions to leverage initial investments in otherwise useful conventional handles and/or fiber-illuminated handles. Universal laryngoscope blades may also reap the benefits of economies of scale, since the universal laryngoscope blades may be produced in greater numbers due to the compatibility with both existing conventional handles and fiber-illuminated handles.

## Claims

1. A universal laryngoscope blade (500, 500') comprising:
a blade body (510) shaped to provide a direct view of a larynx, the blade body including a first end and a second end, the second end being configured for insertion into the larynx;
a viewer (520, 520') connected to the blade body, the viewer being configured to function independently from each of a conventional handle (200) and a fiber-illuminated handle (400); and
a blade fitting (530) disposed at the first end of the blade body, **characterized in that** the blade fitting is removably connectable to, one at a time, each of a hook-on fitting (210) of the conventional handle (200) and a hook-on fitting (410) of the fiber-illuminated handle (400), the hook-on fitting of the conventional handle having at least one physical dimension that is different from at least one physical dimension of the hook-on fitting of the fiber-illuminated handle; and
a power source (528, 600) configured to be in electrical communication with the viewer (520) for supplying power to the viewer when the laryngoscope blade is engaged to one of the conventional handle and the fiber-illuminated handle in an operating position;
wherein the blade fitting (530) includes a body (532) having a thickness (Tu) that is configured to be received within, one at a time, a seat (219) of the hook-on fitting (210) of the conventional handle (200) and a seat (419) of the hook-on fitting (410) of the fiber-illuminated handle (400), the seat of the hook-on fitting of the conventional handle having a width that is less than a width of the seat of the hook-on fitting of the fiber-illuminated handle; and
wherein the blade fitting (530) includes a first electrical contact in electrical communication with the power source and a second electrical contact in electrical communication with the viewer, wherein the first electrical contact is configured to be in electrical communication with the second electrical contact via an electrically conductive hinge pin (218, 418) of one of the conventional handle and the fiber-illuminated handle when the laryngoscope blade is in the operating position.

2. The universal laryngoscope blade according to claim 1, wherein the first electrical contact is in electrical communication with the power source via a first electrical wire (512"), and wherein the second electrical contact is in electrical communication with the viewer via a second electrical wire (514").

3. The universal laryngoscope blade according to claim 1 or claim 2, wherein a bottom of the body (532) of the blade fitting (530) is free of functional elements.

4. The universal laryngoscope blade according to any one of claims 1-3, wherein the blade fitting (530) further includes a hinge slot (534) that extends into the body (532) at an angle from a front of the body towards a central region of the body and that terminates at a semicircular bearing surface (535).

5. The universal laryngoscope blade according to claim 4, wherein the hinge slot (534) is dimensioned to receive in abutment with the semicircular bearing surface (535), one at a time, a hinge pin (218) of the hook-on fitting (210) of the conventional handle (200) and a hinge pin (418) of the hook-on fitting (410) of the fiber-illuminated handle (400), the hinge pin of the hook-on fitting of the conventional handle having a diameter that is less than a diameter of the hinge pin of the hook-on fitting of the fiber-illuminated handle.

6. The universal laryngoscope blade according to claim 5, wherein the blade fitting (530) further includes a hinge lock (536), disposed within a surface of the hinge slot (534), that is configured to lock within the hinge slot, one at a time, the hinge pin (218) of the hook-on fitting (210) of the conventional handle (200) and the hinge pin (418) of the hook-on fitting (410) of the fiber-illuminated handle (400).

7. The universal laryngoscope blade according to any one of claims 1-6, wherein the blade fitting (530) further includes at least one side lock (538a, 538b) provided on a side of the body (532), the at least one side lock is configured to lock the body of the blade fitting within, one at a time, the seat of the hook-on fitting of the conventional handle and the seat of the hook-on fitting of the fiber-illuminated handle.

8. The universal laryngoscope blade according to claim 7, wherein a center point of the at least one side lock (538a, 538b) is spaced rearwardly from a virtual center point of the semicircular bearing surface (535) by a horizontal distance component and spaced below the virtual center point of the semicircular bearing surface by a vertical distance component, the horizontal distance component being 13.0 mm and the vertical distance component being 0.8 mm.

9. The universal laryngoscope blade according to any one of claims 7 and 8, wherein the at least one side lock (538a, 538b) is configured to engage with, one at a time, at least one lock slot (216a, 216b) of the hook-on fitting (210) of the conventional handle (200) and at least one lock slot (416a, 416b) of the hook-on fitting (410) of the fiber-illuminated handle (400).

10. The universal laryngoscope blade according to any one of claims 1-9, wherein the viewer (520, 520') is configured to emit light independently from each of the conventional handle and the fiber-illuminated handle.

11. The universal laryngoscope blade according to any one of claims 1-10, wherein the viewer (520, 520') is configured to capture at least one image independently from each of the conventional handle and the fiber-illuminated handle.

12. The universal laryngoscope blade according to any one of claims 1-11, wherein the viewer (520, 520') includes:
a lamp (524) configured to emit light into the larynx;
a camera (525) configured to capture at least one image of the larynx; and
at least one cable (523) that provides electronic communication between an external device, and the lamp and the camera.

13. The universal laryngoscope blade according to any one of claims 1-12, wherein the viewer (520, 520') includes:
a lamp (524) configured to emit light into the larynx;
a camera (525) configured to capture are least one image of the larynx;
a transceiver (526) configured to wirelessly transmit the at least one image to an external device;
a processor (527) configured to control the lamp, the camera, and the transceiver; and
a power source (528) configured to supply power to the lamp, the camera, the transceiver, and the processor.

14. The universal laryngoscope blade according to any one of claims 1-13, wherein
the hook-on fitting of the conventional handle is dimensioned to conform to ISO standard 7376 for conventional handles and the hook-on fitting of the fiber-illuminated handle is dimensioned to conform to ISO standard 7376 for fiber-illuminated handles.

## Patentansprüche

1. Universeller Laryngoskopspatel (500, 500'), der Folgendes umfasst:
einen Spatelkörper (510), der so geformt ist, dass er eine direkte Ansicht eines Kehlkopfs bereitstellt, wobei der Spatelkörper ein erstes Ende und ein zweites Ende beinhaltet, wobei das zweite Ende zum Einführen in den Kehlkopf ausgelegt ist;
ein Betrachtungsgerät (520, 520'), das mit dem Spatelkörper verbunden ist, wobei das Betrachtungsgerät dazu ausgelegt ist, unabhängig sowohl von einem herkömmlichen Griff (200) als auch von einem faserbeleuchteten Griff (400) zu funktionieren; und
ein am ersten Ende des Spatelkörpers angeordnetes Spatelanschlussstück (530), **dadurch gekennzeichnet, dass** das Spatelanschlussstück immer nur mit einem eines Einrastanschlussstücks (210) des herkömmlichen Griffs (200) und eines Einrastanschlussstücks (410) des faserbeleuchteten Griffs (400) lösbar verbunden werden kann, wobei das Einrastanschlussstück des herkömmlichen Griffs mindestens eine physische Abmessung aufweist, die sich von mindestens einer physischen Abmessung des Einrastanschlussstücks des faserbeleuchteten Griffs unterscheidet; und
eine Leistungsquelle (528, 600), die dazu ausgelegt ist, in elektrischer Verbindung mit dem Betrachtungsgerät (520) zu sein, um dem Betrachtungsgerät Leistung zuzuführen, wenn der Laryngoskopspatel in einer Betriebsposition mit dem herkömmlichen Griff oder mit dem faserbeleuchteten Griff in Eingriff steht;
wobei das Spatelanschlussstück (530) einen Körper (532) mit einer Dicke (Tu) beinhaltet, die dazu ausgelegt ist, dass darin immer nur einer eines Sitzes (219) des Einrastanschlussstücks (210) des herkömmlichen Griffs (200) und eines Sitzes (419) des Einrastanschlussstücks (410) des faserbeleuchteten Griffs (400) aufgenommen wird, wobei der Sitz des Einrastanschlussstücks des herkömmlichen Griffs eine Breite aufweist, die kleiner als eine Breite des Sitzes des Einrastanschlussstücks des faserbeleuchteten Griffs ist; und
wobei das Spatelanschlussstück (530) einen ersten elektrischen Kontakt in elektrischer Verbindung mit der Leistungsquelle und einen zweiten elektrischen Kontakt in elektrischer Verbindung mit dem Betrachtungsgerät beinhaltet, wobei der erste elektrische Kontakt dazu ausgelegt ist, über einen elektrisch leitfähigen Scharnierstift (218, 418) des herkömmlichen Griffs oder des faserbeleuchteten Griffs in elektrischer Verbindung mit dem zweiten elektrischen Kontakt zu sein, wenn sich der Laryngoskopspatel in der Betriebsposition befindet.

2. Universeller Laryngoskopspatel nach Anspruch 1, wobei der erste elektrische Kontakt über einen ersten elektrischen Draht (512") in elektrischer Verbindung mit der Leistungsquelle steht und wobei der zweite elektrische Kontakt über einen zweiten elektrischen Draht (514") in elektrischer Verbindung mit dem Betrachtungsgerät steht.

3. Universeller Laryngoskopspatel nach Anspruch 1 oder Anspruch 2, wobei ein unterster Teil des Körpers (532) des Spatelanschlussstücks (530) frei von Funktionselementen ist.

4. Universeller Laryngoskopspatel nach einem der Ansprüche 1-3, wobei das Spatelanschlussstück (530) ferner einen Scharnierschlitz (534) beinhaltet, der sich in einem Winkel von einer Vorderseite des Körpers zu einem zentralen Bereich des Körpers in den Körper (532) erstreckt und der an einer halbkreisförmigen Lagerfläche (535) endet.

5. Universeller Laryngoskopspatel nach Anspruch 4, wobei der Scharnierschlitz (534) so dimensioniert ist, dass er in Anlage an der halbkreisförmigen Lagerfläche (535) immer nur eines eines Scharnierstifts (218) des Einrastanschlussstücks (210) des herkömmlichen Griffs (200) und eines Scharnierstifts (418) des Einrastanschlussstücks (410) des faserbeleuchteten Griffs (400) aufnimmt, wobei der Scharnierstift des Einrastanschlussstücks des herkömmlichen Griffs einen Durchmesser aufweist, der kleiner als ein Durchmesser des Scharnierstiftes des Einrastanschlussstücks des faserbeleuchteten Griffs ist.

6. Universeller Laryngoskopspatel nach Anspruch 5, wobei das Einrastanschlussstück (530) ferner eine Scharnierverriegelung (536) beinhaltet, die innerhalb einer Oberfläche des Scharnierschlitzes (534) angeordnet ist, die dazu ausgelegt ist, immer nur eines des Scharnierstifts (218) des Einrastanschlussstücks (210) des herkömmlichen Griffs (200) und des Scharnierstifts (418) des Einrastanschlussstücks (410) des faserbeleuchteten Griffs (400) in dem Scharnierschlitz zu verriegeln.

7. Universeller Laryngoskopspatel nach einem der Ansprüche 1-6, wobei das Einrastanschlussstück (530) ferner mindestens eine Seitenverriegelung (538a, 538b) beinhaltet, die auf einer Seite des Körpers (532) bereitgestellt ist, wobei die mindestens eine Seitenverriegelung dazu ausgelegt ist, den Körper des Einrastanschlussstücks in immer nur einem des Sitzes des Einrastanschlussstücks des herkömmlichen Griffs und des Sitzes des Einrastanschlussstücks des faserbeleuchteten Beschlags zu verriegeln.

8. Universeller Laryngoskopspatel nach Anspruch 7, wobei ein Mittelpunkt der mindestens einen Seitenverriegelung (538a, 538b) von einem virtuellen Mittelpunkt der halbkreisförmigen Lagerfläche (535) durch eine horizontale Abstandskomponente nach hinten beabstandet ist und unter dem virtuellen Mittelpunkt der halbkreisförmigen Lagerfläche durch eine vertikale Abstandskomponente beabstandet ist, wobei die horizontale Abstandskomponente 13,0 mm beträgt und die vertikale Abstandskomponente 0,8 mm beträgt.

9. Universeller Laryngoskopspatel nach einem der Ansprüche 7 und 8, wobei die mindestens eine Seitenverriegelung (538a, 538b) dazu ausgelegt ist, immer nur mit einem mindestens eines Verriegelungsschlitzes (216a, 216b) des Einrastanschlussstücks (210) des herkömmlichen Griffs (200) und mindestens eines Verriegelungsschlitzes (416a, 416b) des Einrastanschlussstücks (410) des faserbeleuchteten Griffs (400) in Eingriff zu treten.

10. Universeller Laryngoskopspatel nach einem der Ansprüche 1-9, wobei das Betrachtungsgerät (520, 520') dazu ausgelegt ist, unabhängig sowohl von dem herkömmlichen Griff als auch von dem faserbeleuchteten Griff Licht zu emittieren.

11. Universeller Laryngoskopspatel nach einem der Ansprüche 1-10, wobei das Betrachtungsgerät (520, 520') dazu ausgelegt ist, unabhängig sowohl von dem herkömmlichen Griff als auch von dem faserbeleuchteten Griff mindestens ein Bild aufzunehmen.

12. Universeller Laryngoskopspatel nach einem der Ansprüche 1-11, wobei das Betrachtungsgerät (520, 520') Folgendes beinhaltet:
eine Lampe (524), die dazu ausgelegt ist, Licht in den Kehlkopf zu emittieren;
eine Kamera (525), die dazu ausgelegt ist, mindestens ein Bild des Kehlkopfs aufzunehmen; und
mindestens ein Kabel (523), das eine elektronische Verbindung zwischen einer externen Vorrichtung und der Lampe und der Kamera bereitstellt.

13. Universeller Laryngoskopspatel nach einem der Ansprüche 1-12, wobei das Betrachtungsgerät (520, 520') Folgendes beinhaltet:
eine Lampe (524), die dazu ausgelegt ist, Licht in den Kehlkopf zu emittieren;
eine Kamera (525), die dazu ausgelegt ist, mindestens ein Bild des Kehlkopfs aufzunehmen;
einen Sendeempfänger (526), der dazu ausgelegt ist, das mindestens eine Bild drahtlos an eine externe Vorrichtung zu übertragen;
einen Prozessor (527), der dazu ausgelegt ist, die Lampe, die Kamera und den Sendeempfänger zu steuern; und
eine Leistungsquelle (528), die dazu ausgelegt ist, der Lampe, der Kamera, dem Sendeempfänger und dem Prozessor Leistung zuzuführen.

14. Universeller Laryngoskopspatel nach einem der Ansprüche 1-13, wobei das Einrastanschlussstück des herkömmlichen Griffs so dimensioniert ist, dass es der ISO-Norm 7376 für herkömmliche Griffe entspricht, und das Einrastanschlussstück des faserbeleuchteten Griffs so dimensioniert ist, dass es der ISO-Norm 7376 für faserbeleuchtete Griffe entspricht.

## Revendications

1. Lame de laryngoscope universelle (500, 500') comprenant :
un corps de lame (510) conformé pour fournir une vue directe d'un larynx, le corps de lame comprenant une première extrémité et une seconde extrémité, la seconde extrémité étant configurée pour être insérée dans le larynx ;
un visualiseur (520, 520') relié au corps de lame, le visualiseur étant configuré pour fonctionner indépendamment tant d'un manche conventionnel (200) que d'un manche éclairé par fibre (400) ; et
un raccord de lame (530) disposé à la première extrémité du corps de lame, **caractérisé en ce que** le raccord de lame peut être relié de manière amovible, un à la fois, à chacun d'un raccord d'accrochage (210) du manche conventionnel (200) et d'un raccord d'accrochage (410) du manche éclairé par fibre (400), le raccord d'accrochage du manche conventionnel ayant au moins une dimension physique différente d'au moins une dimension physique du raccord d'accrochage du manche éclairé par fibre ; et
une source d'alimentation (528, 600) configurée pour être en communication électrique avec le visualiseur (520) afin d'alimenter le visualiseur lorsque la lame de laryngoscope est en prise avec l'un du manche conventionnel et du manche éclairé par fibre dans une position de fonctionnement ;
le raccord de lame (530) comprenant un corps (532) ayant une épaisseur (Tu) configurée pour être reçue à l'intérieur, un à la fois, d'un siège (219) du raccord d'accrochage (210) du manche conventionnel (200) et d'un siège (419) du raccord d'accrochage (410) du manche éclairé par fibre (400), le siège du raccord d'accrochage du manche conventionnel ayant une largeur inférieure à une largeur du siège du raccord d'accrochage du manche éclairé par fibre ; et
le raccord de lame (530) comprenant un premier contact électrique en communication électrique avec la source d'alimentation et un second contact électrique en communication électrique avec le visualiseur, le premier contact électrique étant configuré pour être en communication électrique avec le second contact électrique par l'intermédiaire d'une broche de charnière conductrice d'électricité (218, 418) de l'un du manche conventionnel et du manche éclairé par fibre lorsque la lame de laryngoscope est dans la position de fonctionnement.

2. Lame de laryngoscope universelle selon la revendication 1, le premier contact électrique étant en communication électrique avec la source d'alimentation par l'intermédiaire d'un premier fil électrique (512"), et le second contact électrique étant en communication électrique avec le visualiseur par l'intermédiaire d'un second fil électrique (514").

3. Lame de laryngoscope universelle selon la revendication 1 ou la revendication 2, un fond du corps (532) du raccord de lame (530) étant exempt d'éléments fonctionnels.

4. Lame de laryngoscope universelle selon l'une quelconque des revendications 1 à 3, le raccord de lame (530) comprenant en outre une fente de charnière (534) qui s'étend dans le corps (532) selon un angle à partir d'une partie avant du corps vers une région centrale du corps et qui se termine à une surface d'appui semi-circulaire (535).

5. Lame de laryngoscope universelle selon la revendication 4, la fente de charnière (534) étant dimensionnée pour recevoir en butée avec la surface d'appui semi-circulaire (535), une à la fois, une broche de charnière (218) du raccord d'accrochage (210) du manche conventionnel (200) et une broche de charnière (418) du raccord d'accrochage (410) du manche éclairé par fibre (400), la broche de charnière du raccord d'accrochage du manche conventionnel ayant un diamètre inférieur à un diamètre de la broche de charnière du raccord d'accrochage du manche éclairé par fibre.

6. Lame de laryngoscope universelle selon la revendication 5, le raccord de lame (530) comprenant en outre un verrou de charnière (536), disposé à l'intérieur d'une surface de la fente de charnière (534), qui est configuré pour verrouiller à l'intérieur de la fente de charnière, une à la fois, la broche de charnière (218) du raccord d'accrochage (210) du manche conventionnel (200) et la broche de charnière (418) du raccord d'accrochage (410) du manche éclairé par fibre (400).

7. Lame de laryngoscope universelle selon l'une quelconque des revendications 1 à 6, le raccord de lame (530) comprenant en outre au moins un verrou latéral (538a, 538b) prévu sur un côté du corps (532), l'au moins un verrou latéral étant configuré pour verrouiller le corps du raccord de lame à l'intérieur, un à la fois, du siège du raccord d'accrochage du manche conventionnel et du siège du raccord d'accrochage du manche éclairé par fibre.

8. Lame de laryngoscope universelle selon la revendication 7, un point central de l'au moins un verrou latéral (538a, 538b) étant espacé vers l'arrière d'un point central virtuel de la surface d'appui semi-circulaire (535) par une composante de distance horizontale et espacé au-dessous du point central virtuel de la surface d'appui semi-circulaire par une composante de distance verticale, la composante de distance horizontale étant de 13,0 mm et la composante de distance verticale étant de 0,8 mm.

9. Lame de laryngoscope universelle selon l'une quelconque des revendications 7 et 8, l'au moins un verrou latéral (538a, 538b) étant configuré pour venir en prise, une à la fois, avec au moins une fente de verrouillage (216a, 216b) du raccord d'accrochage (210) du manche conventionnel (200) et au moins une fente de verrouillage (416a, 416b) du raccord d'accrochage (410) du manche éclairé par fibre (400).

10. Lame de laryngoscope universelle selon l'une quelconque des revendications 1 à 9, le visualiseur (520, 520') étant configuré pour émettre de la lumière indépendamment tant du manche conventionnel que du manche éclairé par fibre.

11. Lame de laryngoscope universelle selon l'une quelconque des revendications 1 à 10, le visualiseur (520, 520') étant configuré pour capturer au moins une image indépendamment tant du manche conventionnel que du manche éclairé par fibre.

12. Lame de laryngoscope universelle selon l'une quelconque des revendications 1 à 11, le visualiseur (520, 520') comprenant :
une lampe (524) configurée pour émettre de la lumière dans le larynx ;
une caméra (525) configurée pour capturer au moins une image du larynx ; et
au moins un câble (523) qui assure une communication électronique entre, d'une part, un dispositif externe et, d'autre part, la lampe et la caméra.

13. Lame de laryngoscope universelle selon l'une quelconque des revendications 1 à 12, le visualiseur (520, 520') comprenant :
une lampe (524) configurée pour émettre de la lumière dans le larynx ;
une caméra (525) configurée pour capturer au moins une image du larynx ;
un émetteur-récepteur (526) configuré pour transmettre sans fil l'au moins une image à un dispositif externe ;
un processeur (527) configuré pour commander la lampe, la caméra et l'émetteur-récepteur ; et
une source d'alimentation (528) configurée pour alimenter la lampe, la caméra, l'émetteur-récepteur et le processeur.

14. Lame de laryngoscope universelle selon l'une quelconque des revendications 1 à 13, le raccord d'accrochage du manche conventionnel étant dimensionné pour se conformer à la norme ISO 7376 pour les manches conventionnels et le raccord d'accrochage du manche éclairé par fibre étant dimensionné pour se conformer à la norme ISO 7376 pour les manches éclairés par fibre.
